# EUROPEAN PATENT APPLICATION

(11) **EP 1 552 825 A1**
(43) Date of publication of application: **13.07.2005**
(21) Application number: 03730850.9
(22) Date of filing: 05.06.2003
(51) Int. Cl.: A61K 31/18, A61K 31/4725, A61K 45/00, A61P 13/00, A61P 13/02, A61P 13/10, A61P 43/00, C07D 453/02

(54) **THERAPEUTIC AGENT FOR OVERACTIVE BLADDER**

(30) Priority: 07.06.2002 JP 2002166408
(71) Applicant: YAMANOUCHI PHARMACEUTICAL CO. LTD., Tokyo 103-8411 (JP)
(72) Inventor: VAN MEETEREN, Rian, c/o Yamanouchi Europe B.V, NL-2350 AC Leiderdorp (NL); VISSER, Nico j., c/o Yamanouchi Europe B.V, NL-2350 AC Leiderdorp (NL); KAJII, Hiroshi, c/o Yamanouchi Pharma. Co.Ltd, Tokyo 174-8612 (JP); TAKIGUCHI, Nobuyuki, c/o Yamanouchi Pharma. Co.Ltd, Tokyo 174-8612 (JP)
(74) Representative: HOFFMANN EITLE
(86) International application number: PCT/JP2003/007149
(87) International publication number: WO 2003/103659

(57) **Abstract**

A therapeutic agent for overactive bladder containing tamsulosin or a pharmaceutically acceptable salt thereof as an effective ingredient.

## Description

### Technical Field of the Invention

The present invention relates to a drug, particularly an overactive bladder treating drug.

### Background Art

Lower urinary tract dysfunction is broadly classified into urinary collection disorder (increased urinary frequency and urinary incontinence) and urinary disorder (difficulty in urination and anuresis). Urinary incontinence is a typical example of urinary collection disorder, and defined as urine leakage demonstrable subjectively, thereby giving difficulties both in leading a normal daily life and in view of hygiene. Increased urinary frequency is a state exceeding normal frequency of urination and defined as:approximately 2 times or more at night and 9 times or more during a 24 hour period. Urinary incontinence is classified into abdominal pressure urinary incontinence that takes place when abdominal pressure is given such as during coughing or sneezing, impending urinary incontinence that takes place suddenly, with urine leakage before a patient reaches a toilet and mixed-type urinary incontinence of abdominal pressure urinary incontinence with impending urinary incontinence (Current Therapy, 19 (12), 8-11, 2001).

Overactive bladder is a medical condition referring to the symptoms of frequency and urgency, with or without urge incontinence, when appearing in the absence of local pathological, neurological or metabolic factors that would account for these symptoms [Urology, 55 (suppl. 5A)', p1-2, 2000] . To be more specific, the following 3 groups are included in over-active bladder :(1)increased urinary frequency and urinary urgency alone, (2) increased urinary frequency, urinary urgency and impending urinary incontinence and (3)mixed-type urinary incontinence.

Drugs for treating an overactive bladder include the muscarinic receptor antagonists that can reduce the contraction of bladder detrusor muscle. The muscarinic receptor is abundantly present in the bladder detrusor muscle and triggers contraction by stimulating the bladder detrusor muscle.

Studies on new action mechanisms are now in progress about K channel activators, βₐ receptor agonists, drugs suppressing central urinary reaction, and prostaglandin synthesis inhibitors, in addition to muscarinic receptor antagonists (Medicine and Drug Journal, 38 (S-1), 283-288, 2002).

α₁ receptor is abundantly present in the bottom of the bladder, urinary tract, prostate, and epithelial membrane of the prostate, and stimulates the smooth muscles to cause contraction, thus increasing urethral resistance. Thus, α₁ receptor blockers that reduce the urethral resistance are considered effective in treating urinary disorder. Further, α₁ receptor agonists that increase the urethral resistance are considered effective in treating urinary collection disorder. Forexample, WO96/38143 has described that α_{1A} receptor selective agonists are effective in treating urinary incontinence on the basis of the fact that α_{1A} receptor is involved in the contraction of the urinary tract and bladder neck. WO99/57131 has described that α_{1D} receptor blockers are effective in treating the stimulus symptoms found in disorders of the bladder and lower urinary tract on the basis of the fact that α_{1D} receptor is locally present in the bladder.

Now, new drugs for treating an overactive bladder have been greatly anticipated.

### Disclosure of the Invention

Under these circumstances, the inventor has found that tamsulosin or its salt is effective in treating an overactive bladder.

More particularly, the present invention relates to a pharmaceutical composition for the therapy of overactive bladder where said composition contains tamsulosin or a pharmaceutically acceptable salt thereof as an effective ingredient.

The present invention further relates to the use of tamsulosin or a pharmaceutically acceptable salt thereof for the manufacture of a therapeutic agent for overactive bladder. The present invention furthermore relates to a method for the therapy of overactive bladder where said method includes administration of therapeutic effective amount of tamsulosin or a pharmaceutically acceptable salt thereof to a patient.

The chemical name of tamsulosin is (R) (-)-5-[2-[[2-(o-ethoxyphenoxy)ethyl]amino]propyl]-2-met hoxybenzene-sulfonamide and is represented by the following structural formula. Tamsulosin has been firstly disclosed in the Japanese Patent Laid-Open No. Sho-56/110665 (corresponding European patent publication No.34432) together with its pharmaceutically acceptable salts.

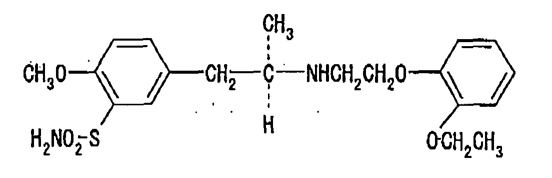

It has been known that tamsulosin and its salt exhibit adrenaline α_{1A} receptor blocking actions, and its hydrochloride (tamsulosin hydrochloride), in particular, acts to block α₁ receptor in the urinary tract and prostate, thus finding a wide use as a drug for improving urinary disorder associated with prostate hypertrophy by reducing the prostate pressure part of urethral pressure profile. In recent years, these substances have been also demonstrated to be effective in treating urinary disorder associated with neurogenic bladder and lower urinary tract disorders (urinary disorder in association with functional closure of the lower urinary tract and not in association with clear organic disorder or neurological abnormality in the lower urinary tract) (WO00/00187 (corresponding European patent publication No.1088551), WO001/10436 (corresponding European patent publication No.1203582)).

No reports have been so far available that have confirmed the efficacy against urinary collection disorder (increased urinary frequency, urinary incontinence). However, the present inventor has confirmed for the first time the clinical efficacy of tamsulosin hydrochloride in treating an overactive bladder.

The present invention further relates to the use of tamsulosin or a pharmaceutically acceptable salt thereof for the manufacture of a medicament to be used in combination with muscarinic receptor antagonist in the treatment of overactive bladder. The present invention furthermore relates to a method for the therapy of overactive bladder where said method includes administration of therapeutically effective amount of tamsulosin or a pharmaceutically acceptable salt thereof to a patient in combination with therapeutically effective amount of muscarinic receptor antagonist.

Further, the invention relates to a pharmaceutical composition for the therapy of overactive bladder where said composition contains tamsulosin or a pharmaceutically acceptable salt thereof and muscarinic receptor antagonist or its pharmacologically permissible salt as active ingredients. Tamsulosin (α₁ receptor blocker) and muscarinic receptor antagonist are confirmed to provide synergetic effects due to different action mechanisms.

The invention will be explained in further detail as follows:

In the present invention, the term "overactive bladder" stands for a medical condition referring to the symptoms of frequency and urgency, with or without urge incontinence, when appearing in the absence of local pathological, neurological or metabolic factors that would account for these symptoms. The patients of overactive bladder include not only adults, but also children.

A therapeutic agent for overactive bladder is a pharmaceutical agent which treats overactive bladder and/or a pharmaceutical agent which improves the symptom of overactive bladder. A pharmaceutical composition for the therapy of overactive bladder contains effective ingredients which treat overactive bladder and/or improve the symptom of overactive bladder and pharmaceutically acceptable carriers thereof.

Tamsulosin and its pharmaceutically acceptable salt are easily available by means of the methods described in the Japanese Patent Laid-Open Nos. Sho-56/110665 (corresponding European patent publication No.34432) and Sho-62/114952 (corresponding Canadian patent No.1282077) or the methods similar thereto.

Tamsulosin is able to form pharmaceutically acceptable acid- and base-addition salts with a wide range of inorganic and organic acids or bases. Such salts also constitute a part of the present invention. Their examples are salts with inorganic acids such as hydrochloric acid, sulfuric acid, phosphoric acid; salts with organic acids such as fumaric acid, malic acid, citric acid, succinic acid; salts with alkaline metals such as sodium, potassium; and salts with alkaline earth metals such as calcium, magnesium, etc. In the present invention, hydrochlorides are most preferred. Such salts-can be manufactured by a conventional method.

The pharmaceutical agent of the present invention can be prepared as oral solid preparations, oral liquid preparations or injection preparations by a conventional method using organic or inorganic carrier, filler and other additives suitable for oral or parenteral administration. Preferred ones are oral solid preparations which can be easily administered by a patient himself/herself and are convenient for preservation and carrying and, to be more specific, they are tablets, diluted powder, granules, fine granules, capsules, pills, etc.

In such solid preparations, the active substance is mixed with at least one inert diluent such as lactose, mannitol, glucose, microcrystalline cellulose, starch, and polyvinylpyrrolidone or magnesium metasilicate aluminate. The composition may contain additives other than the inert diluent according to a conventional method and their examples may be binders such as hydroxypropyl cellulose and hydroxypropylmethyl cellulose; lubricants such as magnesium stearate, calcium stearate, polyethylene glycol, starch and talc; disintegrants such as calcium cellulose glycolate; stabilizers such as lactose; solubilizing aids such as glutamic acid and aspartic acid; plasticizers such as Tween 80 and triacetin; and coloring agents such as titanium oxide and iron sesquioxide. If necessary, tablets or pills may be coated with a sugar coat or with a film of a gastric or an enteric substance such assucrose, gelatin, agar, pectin, hydroxypropyl cellulose and hydroxypropylmethyl cellulose Phthalate.

The most preferred preparation in the present invention is a sustained-release preparation of a sustained releasing type. The sustained-release preparation may be made into tablets, granules, fine granules or capsules by a known method. The sustained-release preparation is prepared by coating tablets, fine granules, fine granules or capsules with, for example, fat/oil, fatty acid ester of polyglycerol, hydroxypropyl cellulose, etc. by a known method.

The sustained-release preparation disclosed in the Japanese Patent Laid-Open No. Sho-62/9 (corresponding European patent publication No. 194838) is particularly preferred. Thus, in each unit preparation, particles which are prepared by granulation after adding an elution suppressor to a mixture of an active compound and not less than 50% by weight of a unit-forming substance in a unit are filled in a capsule to prepare a capsule preparation or to prepare a tablet by a conventional method. Crystalline cellulose is preferable as the unit-forming substance. With regard to the elution suppressor, a water-insoluble high-molecular substance such as an acrylic polymer, copolymer or cellulose derivative is used and it is appropriate that the elution suppressor is used, for example, in a form of an aqueous suspension, an aqueous emulsion or a solution in a water-containing organic solvent. Examples of the commercially available one are Eudragit L 30D55 (methacrylic acid copolymer LD), Eudragit E 30D (emulsion of copolymer of ethyl acrylate with methyl methacrylate) and Aquacoat ECD-30 (aqueous suspension of ethyl cellulose) and they may be used as elution suppressors either as they are or after diluting with water if necessary.

In the intention, the most preferable is an orally sustained-release preparation with prolonged action that is able to release a certain active ingredient for a prolonged period of time of 12 - 24 hours after oral administration through favorable drug release not only in the upper digestive tract but also in the lower digestive tract.

For example, the orally sustained-release preparation with prolonged action disclosed in WO94/06414 (corresponding European patent publication No.661045) is able to release an active ingredient even at a colon low in water content because the preparation can well absorb water contents inside the preparation while remaining in the upper digestive tracts and travel down the lower digestive tract in an almost completely gelatinized form. Specifically, this is a hydrogel sustained-release preparation type tablet with a gelatinization rate of 70% or greater but less than 100%, which is obtained by mixing (1) drugs, (2) additves for causing water intrusion into the preparation at a range of 5 ~ 80 % by weight or greater with regard to the preparation as a whole in a solubility by which not more than 5mL of water is necessary for dissolving 1 gram of the preparation and (3) a high-molecular weight substance weighing 70mg or more per one tablet and forming hydrogel at 10 - 95 % by weight with regard to the preparation as a whole, with mean molecular weight of 2, 000, 000 or greater or the viscosity of 1000cps or greater in 1% aqueous solution at 25°C. The additive for causing water intrusion into the preparation includes polyethylene glycol, polyvinyl pyrrolidone , D-sorbitol, xylitol, refined sugar, anhydrous maltose, D-fructose, dextran, glucose, polyoxyethylene polyoxypropylene glycol, sodium chloride, magnesium chloride, citric acid, tartaric acid, glycine, β-alaine, lysine hydrochloride and meglumine. The hydrogel-forming high-molecular substance includes polyethylene oxide, hydroxyproply methylcellulose, carboxymethylcellulose sodium, hydroxyethy cellulose and carboxyvinyl polymer. Further, where polyethylene oxide is used as a hydrogel-forming high-molecular substance, yellow iron sesquioxide and/or red iron sesquioxide are mixed to prevent change in the sustained-release property of tamsulosin even in storage under irradiation, as disclosed in WO01/10466.

Dose of tamsulosin or a pharmaceutically acceptable salt thereof is appropriately decided for each case taking administering route, symptom of the disease, age and sex of the object to be treated, etc. into consideration. Taking into consideration the effects of Test Examples described after, in the case of an oral administration, tamsulosin hydrochloride is administered to an adult usually in a dose of about 0.1 to 2.0 mg/day or, most preferably, in a dose of 0.25 to 1.5 mg/day and that is administered per os after meals once daily.

Though the drug of the invention is sufficiently effective for monotherapy, synergic effects can be obtained by the co-administration of muscarine antagonists having a different action mechanism at the same time or at intervals. A combination drug comprising tamsulosin or its salt, and a muscarinic receptor antagonist can be administered to patients. Muscarinic receptor antagonists include oxybutynin, tolterodine, darifenacin, nubenzepin, zamiphenacine, tiotropium, albamerin, trospium, phesoterozine, temiverine, qunuclidin-3'-yl-1-phenyl1,2,3, 4-tetrahydroisoquinoline-2-carboxylate(solifenacin), 4 (2-methyl-1H-imidazolyl-1-yl)-2,2-diphenylbutyl amide, N-[1-(6-aminopyridine-2-ylmethyl)piperidine-4-yl]-2(R)-[3, 3-difluoro-1(R)-cyclopentyl]-2-hydroxyl-2-phenyl acetoamide and their salts. Preferable are M3 receptor-selective antagonists and particularly preferable are succinic acid of (+)-(1S, 3'R)-qunuclidin-3'-yl 1-phenyl-1, 2, 3, 4-tetrahydroisoquinoline-2-carboxylate (solifenacin).

Solifenacin and its pharmaceutically acceptable salt are easily available by means of the methods described in WO96/20194 or the methods similar thereto. In the case of an oral administration, they are administered to an adult usually in a dose of about 1 to 100 mg/day or, most preferably, in a dose of 5 to 50 mg/day once or dividedly twice.

### Brief Explanation of the Drawings

Fig. 1 shows effect of tamsulosin on contraction before micturition of overactive bladder model rat(A: frequency of contraction; B: amplitude of contraction) (*:p<0.05, **:p,0.01);
Fig. 2 shows effect of tamsulosin sole or combination with solifenacin on contraction before micturition of overactive bladder model rat (A: frequency of contraction; B: amplitude of contraction) (**:p<0.01);

### Best Mode for Carrying Out the Invention

The present invention will now be illustrated in more detail by way of Examples and Test Examples as hereunder although the present invention is not limited to those Examples, etc.

### Example 1.

5 g of tamsulosin hydrochloride and 470 g of crystalline cellulose were well mixed, 500 g of a mixture of 83.3 g of Eudragit L30D-55 (25 g as a solid) with water were added thereto and the mixture was granulated using a high-speed stirring granulator. The resulting particles were in a spherical shape having a particle size of 0.1-1.5 mm where most of them were 0.2-1.0 mm.

The resulting particles were mixed with talc and magnesium stearate and filled in capsules to prepare capsule preparations (containing 0.2 mg of tamsulosin hydrochloride in a capsule).

### Examples 2-6.

The same process as in Example 1 was conducted whereby the particles manufactured according to the formulations of Table 1 were made into capsule preparations.

**Table 1 (unit: grams)**

| Example Number | Tamsulosin Hydrochloride (g) | Crystalline Cellulose (g) | Eudragit L30D-55 (Solid Content) (g) |
|---|---|---|---|
| 2 | 5 | 445 | 166.6 (50) |
| 3 | 5 | 395 | 333.3 (100) |
| 4 | 5 | 482.5 | 41.7(12.5) |
| 5 | 2.5 | 472.5 | 83.3 (25) |
| 6 | 1.25 | 473.75 | 83.3 (25) |

### Example 7.

5 g of tamsulosin hydrochloride, 420 g of crystalline cellulose and 50 g of magnesium stearate were well mixed, a mixture(500g) of 83.3 g of Eudragit L30D-55 (25 g as a solid) with water were added thereto and the mixture was kneaded followed by granulating by a centrifugal fluid granulator. The resulting particles were in a spherical shape having a particle size of 0.1-1.5 mm where most of them were 0.2-1.0 mm.

The resulting particles were mixed with talc and magnesium stearate and filled in capsules to prepare capsule preparations (containing 0.2 mg of tamsulosin hydrochloride in a capsule).

### Examples 8-10.

The same process as in Example 7 was conducted whereby the particles manufactured according to the formulations of Table 2 were made into capsule preparations.

**Table 2 (unit: grams)**

| Example Number | Tamsulosin Hydrochloride | Crystalline Cellulose | Magnesium Stearate | Eudragit L30D-55 (Solid Content) |
|---|---|---|---|---|
| 8 | 5 | 460 | 10 | 83.3 (25) |
| 9 | 5 | 445 | 25 | 83.3 (25) |
| 10 | 2.5 | 462.5 | 10 | 83.3(25) |

### Example 11.

80 g of hydrogenated castor oil was melted, 10 g of tamsulosin hydrochloride and 30 g of hydroxypropyl cellulose having a low degree of substitution were dispersed therein and the dispersion was made into fine particles by means of a spray condyling. The resulting fine particles (60 g) were well mixed with 440 g of crystalline cellulose, 500 g of water were added thereto and the mixture was granulated using a centrifugal fluid granulator.

The resulting particles were mixed with talc and magnesium stearate and filled in capsules to prepare capsule preparations.

### Example 12.

| | |
|---|---|
| Tamsulosin hydrochloride | 0.2 (mg) |
| D-sorbitol | 17.8 |
| Polyethylene oxide (Polyox WSR N-60K) | 180 |
| Smoothing agent | 2 |

Tamsulosin hydrochloride, D-sorbitol and polyethylene oxide (Polyox WSR N-60) were subjected to ethanol wet type granulation, using ethanol, and then dried. A smoothing agent was added to the dried product, mixed and tabulated to obtain an orally sustained-release preparation with prolonged action (8mm across) weighing 200mg.

### Example 13.

An orally sustained-release preparation with prolonged action was prepared according to the following prescription by the method similar to that used in Embodiment 12.

**Table 3**

| |
|---|
| Tamsulosin hydrochloride |
| Polyethylene glycol 8000 |
| Polyethylene oxide (Polyox WSR 303) |
| Magnesium stearate |
| 0.25mg tablet |
| 0.5mg tablet |
| 1.0mg tablet |

### Example 14

The following ingredients are mixed and filled in capsules to prepare capsule preparation.

| | 1mg capsule | 10mg capsule | 100mg capsule |
|---|---|---|---|
| solifenacin succinate | 1.0mg | 10.0mg | 100.0mg |
| lactose | 199.0mg | 190.0mg | 100.0mg |
| total | 200.0mg | 200.0mg | 200.0mg |

### Test Example 1. Clinical Test to Patients Suffering from overactive bladder

A clinical study was conducted in response to patients with overactive bladder under the following conditions.
Subjects : Female patients aged 18 - 70 who have exhibited symptoms of an over-active bladder (increased urinary frequency, urinary urgency and impending urinary incontinence) for more than 3 months.
Investigational product and method of administration : the orally sustained-release preparation with prolonged action as prepared in Embodiment 13 (0.25mg tablet, 0.5mg tablet or 1mg tablet) was orally administered in the morning once daily. Patients who were to receive 1.5mg of tamsulosin hydrochloride were given 2 tablets, namely, 0.5mg and 1mg. All the patients were examined by a physician prior to the administration, and 2 weeks, 4 weeks and 6 weeks after the administration.
Duration of treatment : 6 weeks
Items to be observed: The following items were measured and evaluated.

(1) Patient diary
   Patients kept a diary about the frequencies of urination, urinary urgency and impending urinary incontinence as well as the time of getting up in the morning and of going to bed at night for 3 days prior to each examination. They also recorded daily urinary output for 2 days before each examination.
(2) Cystometry
   Some of the patients were measured for intravescial pressure with a cystometer before and after administration.
(3) King health examination form
   Patients were surveyed for the following matters during each medical examination.
   ① "How is your condition of health?"
      [Very good, good, fair, bad, very bad]
   ② "How seriously does the urination problem affect your health?"
      [Not affected at all, slightly affected, fairly affected, very much affected]
   ③ To what degree do you detect the following symptoms ? [Slightly, fairly, seriously]
      1) increased urinary frequency:going to toilet frequently
      2) night urination : awakening for the toilet at night
      3) urinary urgency : inability to control a desire for urination
      4) impending urinary incontinence : leakage associated with a strong desire for urination
      5) abdominal pressure urinary incontinence : leakage due to activities of coughing, sneezing and running
      6) nocturnal enuresis : wetting at night
      7) urinary incontinence at sexual intercourse : leakage at sexual intercourse
      8) urinary tract infection
      9) cystalgia
      10) difficulty in urination
      11) others
   ④ Restrictions on business and domestic affairs
      "How seriously does the problem of urination affect domestic affairs such as cleaning and shopping ? "
      [Not affected at all, slightly affected, fairly affected, very much affected]
      "Does the problem of urination affect business and daily activities other than family chores?
      [Not affected at all, slightly affected, fairly affected, very much affected]
   ⑤ Physical/social restrictions
      "Does the problem of urination affect walking, running, sports, participation in physical exercise? "
      [Not affected at all, slightly affected, fairly affected, very much affected]
      "Does the problem of urination affect travel?"
      [Not affected at all, slightly affected, fairly affected, very much affected]
      "Does the problem of urination affect socializing activities ? "
      [Not affected at all, slightly affected, fairly affected, very much affected]
      "Does the problem of urination affect socializing with your friends ? "
      [Not affected at all, slightly affected, fairly affected, very much affected]
   ⑥ Personal human relationship
      "Does the problem of urination affect relationships with your partner ? "
      [Not affected at all, slightly affected, fairly affected, very much affected]
      "Does the problem of urination affect your sexual activity?"
      [Not affected at all, slightly affected, fairly affected, very much affected]
      "Does the problem of urination affect your family life ? "
      [Not affected at all, slightly affected, fairly affected, very much affected]
   ⑦ Problems of mind
      "Are you depressed by the problem of urination?"
      [Not at all, slightly, fairly, substantially]
      "Are you easily angered or nervous by the problem of urination ? "
      [Not at all, slightly, fairly, substantially]
      "Are you obsessed with self-contempt by the problem of urination ? "
      [Not at all, slightly, fairly, substantially]
   ⑧ Sleeping and vitality.
      "Does the problem of urination affect sleeping ?"
      [Not at all, sometimes, often, always]
      "Do you feel tired?"
      [Not at all, sometimes, often, always]
   ⑨ "Does your condition fall under any of the following? If so, how is your condition?"
      Wearing of urinary pads [No, sometimes, often, always]
      Being careful about intake of drinks [No, sometimes, often, always]
      Changing wet underwear [No, sometimes, often, always]
      Worried about the possibility of odors [No, sometimes, often, always]
      Embarrassed by the problems of urination [No, sometimes, often, always]
(4) Blood and urine were collected during each examination to analyze the following items.
   ① Hematology
      Hemoglobin, hematocrit
   ② Biological chemistry
      Potassium, sodium, urea, creatinine, calcium, albumin, total protein, alkaline phosphatase, γ-GT, aspartate amino transferase, alanine aminotransferase, lactate dehydrogenase, bilirubin, creatinine kinase, total cholesterol and glucose
   ③ Hormone
      Plasma prolactin
   ④ Urinalysis
      Protein, blood, pH, glucose, white blood cell, urobilinogen, bilirubin, ketone, nitrogen
   ⑤ Cultivation of urine (before and at completion of administration)
   ⑥ Pregnancy test (before administration and follow-up period)
   ⑦ Pharmacokinetic study
      Blood concentration of tamsulosin

### Test Example 2. improvement of the irritating symptoms in the urinary bladder of animal model for disease

### [Method].

### (1) Preparation of overactive bladder model rats

Wistar female rats (6 to 7 weeks old) were employed for the preparation. A catheter was inserted into the urinary bladder through the external urethral orifice under anesthesia with sodium pentobarbital in rats. After subjecting the abdominal region to the median section, the proximal urethra was ligated together with the catheter at 2 sites by a silk blade (5-0), and the catheter was removed.

### (2)Cystometry under nonanesthesized condition

### (Measurement procedure)

At more than 6 weeks after surgery, the silk blade used for ligation of the urethra was removed under anesthesia with sodium pentobarbital. A catheter for infusion of physiological saline and for the measurement of the intravesical pressure was inserted into the urinary bladder through the top part of the urinary bladder and fixed. A catheter for administration of a drug was inserted into the jugular vein, and the rat was returned to a housing cage after recovery. At 2 days after surgery, the catheter inserted into the urinary bladder was connected to a syringe pump for continuous infusion of physiological saline (7-20 mL/hr) to induce the micturition reflex and to a pressure transducer to measure the intravesical pressure with a three way stopcock. After the micturition reflex was stabilized, physiological saline or a drug was administered through the catheter for administration of a drug inserted into the jugular vein.

### (Parameters evaluated)

As the parameters evaluated, the frequency of contraction before micturition (the number of contractions for 10 min before starting of micturition), contraction pressure (mean contraction pressure for 10 min before starting of micturition), micturition pressure and interval of micturition were employed for the present test. The effect of a drug was evaluated on the basis of the inhibition ratio against the pre-dosing values (prolongation ratio in the micturition interval).

### (Drug)

Tamsulosin hydrochloride was administered intravenously in increasing the doses at a common ratio of about 3 (1, 3 and 10 µg/kg) . The action of tamsulosin hydrochloride (3 µg/kg) in combination with Solifenacin succinate (3 mg/kg) on each parameter was also evaluated.

### (3)Statistical analysis

Each test was performed at n=5, and the results are expressed as mean ± standard error. Student's t-test and Tukey's test were used for comparison between 2 groups and among multiple groups, respectively.

### [Results]

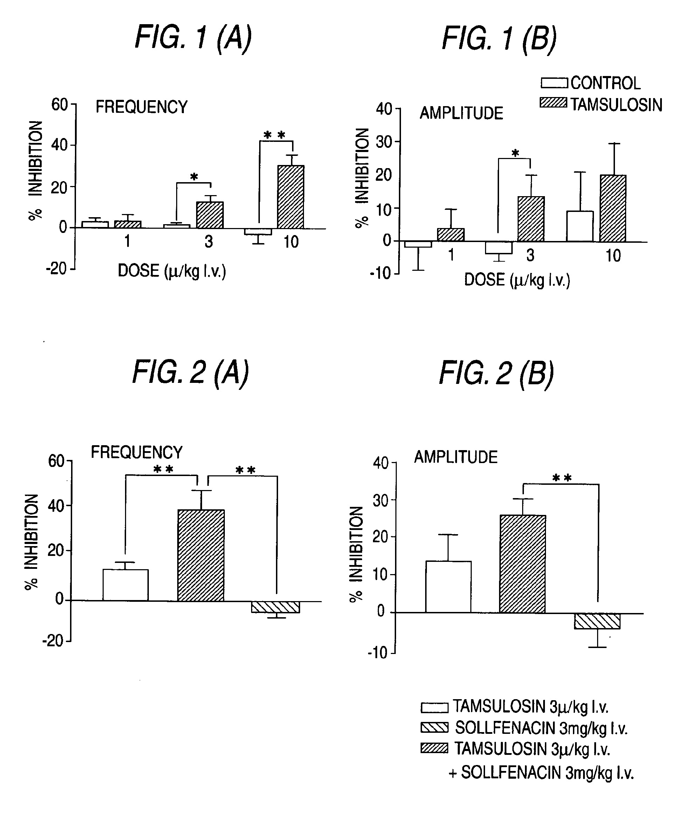

In the BOO rats, the frequency of contraction and contraction pressure before micturition were inhibited in a dose-dependent manner by intravenous administration of tamsulosin hydrochloride (1,3 and 10 µg/kg) (Fig. 1A and B). In intravenous administration of tamsulosin hydrochloride (10 µg/kg), the interval of micturition was prolonged by 9.2%, but there was no influence on the micturition pressure. These results indicated that tamsulosin hydrochloride improved the irritating symptoms in the urinary bladder, suggesting that tamsulosin hydrochloride is probably useful for the treatment of overactive urinary bladder.

When tamsulosin hydrochloride (3 µg/kg i.v.) was administered to BOO rats in combination with solifenacin succinate (3mg/kg i.v.), the inhibitory effect on the frequency of contraction and contraction pressure before micturition were increased compared with that in administration of each drug alone, indicating that the synergistic effect was produced in combination of tamsulosin hydrochloride and solifenacin (Fig. 2A and B). Also, the interval of micturition was prolonged only by 2.1% or 2.6% in administration of tamsulosin hydrochloride (3 µg/kg i.v.) or solifenacin succinate (3 mg/kg i.v.), respectively, but by 13.1% in the combination of tamsulosin hydrochloride and solifenacin succinate. On the other hand, the combination of tamsulosin hydrochloride and solifenacin succinate gave no effect on the micturition pressure similar to administration of tamsulosin hydrochloride alone. Thus, administration in the combination of tamsulosin hydrochloride and solifenacin succinate improved more strongly the irritating symptoms in the urinary bladder compared with administration of tamsulosin hydrochloride alone. Therefore, the combination of tamsulosin hydrochloride and solifenacin succinate will be a useful treatment method for overactive urinary bladder.

### Industrial Applicability

The present invention is able to provide an excellent drug for treating an overactive bladder.

## Claims

1. A pharmaceutical composition for the therapy of overactive bladder where said composition contains tamsulosin or a pharmaceutically acceptable salt thereof as an effective ingredient.

2. A pharmaceutical composition for the therapy of overactivebladder according to claim 1 inwhich said composition contains tamsulosin hydrochloride.

3. A pharmaceutical composition for the therapy of overactive' bladder according to claim 1 or Claim 2 in which said composition contains muscarinic receptor antagonist.

4. A pharmaceutical composition for the therapy of overactive bladder according to claim 3 in which said composition contains qunuclidin-3'-yl 1-phenyl-1, 2, 3, 4-tetrahydroisoquinoline-2-carboxylate or its salt as an effective ingredient.

5. The use of tamsulosin or a pharmaceutically acceptable salt thereof for the manufacture of a therapeutic agent for overactive bladder.

6. The use of tamsulosin or a pharmaceutically acceptable salt thereof for the manufacture of a medicament to be used in combination with muscarinic receptor antagonist in the treatment of overactive bladder.

7. A method for the therapy of overactive bladder where said method includes administration of therapeutically effective amount of tamsulosin or a pharmaceutically acceptable salt thereof to a patient.

8. A method for the therapy of overactive bladder where said method includes administration of therapeutically effective amount of tamsulosin or a pharmaceutically acceptable salt thereof to a patient in combination with therapeutically effective amount of muscarinic receptor antagonist.
